**Europäisches Patentamt**

**European Patent Office**

**Office européen des brevets**

(19)

(11) Veröffentlichungsnummer: **0 010 282**

**A1**

(12)

# EUROPÄISCHE PATENTANMELDUNG

(21) Anmeldenummer: **79103958.9**

(22) Anmeldetag: **15.10.79**

(51) Int. Cl.³: **C 07 C 67/05**
**C 07 C 69/16**

(30) Priorität: **21.10.78 DE 2845974**

(43) Veröffentlichungstag der Anmeldung:
**30.04.80 Patentblatt 80/9**

(84) Benannte Vertragsstaaten:
**BE DE FR GB IT NL**

(71) Anmelder: **BASF Aktiengesellschaft**
**Carl-Bosch-Strasse 38**
**D-6700 Ludwigshafen(DE)**

(72) Erfinder: **Weitz, Hans-Martin, Dr. Dipl.-Chem.**
**Auf dem Koeppel 40**
**D-6702 Bad Duerkheim 1(DE)**

(72) Erfinder: **Hartig, Juergen, Dr. Dipl.-Chem.**
**In der Schleit 9**
**D-6718 Gruenstadt(DE)**

(72) Erfinder: **Platz, Rolf, Dr. Dipl.-Chem.**
**Hansastrasse 5**
**D-6800 Mannheim 1(DE)**

(54) **Verfahren zur Herstellung von Carbonsäureestern vicinaler Glykole.**

(57) Herstellung von Carbonsäureestern vicinaler Glykole durch Oxidation von Olefinen mit Sauerstoff und in Gegenwart von Carbonsäuren sowie von Halogen und von Tellursalzen, indem man als Halogen Jod verwendet und die Konzentration des Wassers im Reaktionsgemisch unter 1 Gew.% hält.

EP 0 010 282 A1

BASF Aktiengesellschaft                    O.Z. 0050/033472

Verfahren zur Herstellung von Carbonsäureestern vicinaler Glykole

Die vorliegende Erfindung betrifft ein verbessertes Verfahren zur Herstellung von Carbonsäureestern vicinaler Glykole durch katalytische Oxidation von Olefinen mit Sauerstoff und in Gegenwart von Carbonsäuren in der Flüssigphase.

Es ist allgemein bekannt, Olefine in der Flüssigphase mit Sauerstoff und Carbonsäuren in Gegenwart von Halogenen und Tellursalzen zu den Carbonsäure- Mono- und Diestern der dem Olefin entsprechenden vicinalen Glykole umzusetzen.

Nach der Lehre der DE-OS 2 038 781 soll hierbei der Wassergehalt im Reaktionsmedium unter 15 Gew.-% gehalten werden, um dadurch die Selektivität der Reaktion zu verbessern. Andererseits wurde festgestellt (DE-OS 2 134 145), daß die Reaktionsgeschwindigkeit mit geringer werdenden Wasserkonzentrationen, vor allem bei Konzentrationen unter 1 Gew.-% und besonders unter 0,5 Gew.-%, stark abnimmt.

Hieraus folgt, daß Wasser einen wesentlichen Einfluß auf die Reaktion hat, indem es sie teils begünstigt und teils beeinträchtigt. Aufgrund der Vorschriften in den beiden

0010282

Öffenlegungsschriften liegt der wirtschaftlich insgesamt optimale Wassergehalt daher zwischen 0,5 und 15 Gew.-% des Reaktionsmediums.

Um welchen genauen Wert es sich hierbei je nach den Reaktionsbedingungen im Einzelfall auch handeln mag, so ist es doch unbefriedigend, daß ein solcher Wert stets einen Kompromiß darstellt.

Der Erfindung lag daher die Aufgabe zugrunde, das in Rede stehende Verfahren dahingehend zu verbessern, daß man zur Erzielung hoher Selektivitäten hinsichtlich der gewünschten Verfahrensprodukte keine Verringerung der Reaktionsgeschwindigkeit in Kauf nehmen muß.

Es wurde gefunden, daß man Carbonsäureester vicinaler Glykole durch Oxidation von Olefinen mit Sauerstoff und in Gegenwart von Carbonsäuren sowie von Halogen und von Tellursalzen in hoher Selektivität und in hohen Raum-Zeit-Ausbeuten erhält, wenn man

- als Halogen Jod verwendet und
- die Konzentrationen des Wassers im Reaktionsgemisch unter 1 Gew.-% hält.

Dieses Verfahren ist insofern bemerkenswert, als die aus den beiden genannten Offenlegungsschriften hervorgehende Gesetzmässigkeit, den Wassergehalt zweckmäßigerweise auf eine Konzentration von 0,5-15 Gew.-% einzustellen, nur für die dort ausschließlich beschriebenen Fälle der Verwendung von Chlor oder Brom im Katalysatorsystem gültig ist, nicht aber für Jod.

Besondere Bedeutung hat die erfindungsgemäße Verfahrensverbesserung für die Umsetzung von Äthylen oder Propylen mit Essigsäure, da die hierbei entstehenden Ester

0010282

des Äthylenglykols bzw. des Propylenglykols unter Abspaltung von Essigsäure in Äthylenoxid bzw. Propylenoxid überführt werden können. Das Verfahren läßt sich jedoch allgemein auf jegliche Olefine anwenden, wobei es von praktischer Bedeutung für $C_2-C_{30}$-Olefine, vor allem aber für $C_2-C_5$-Olefine, ist. Hierbei reagieren 1-Olefine, z.B. But-1-en nach bekannten Gesetzmäßigkeiten leichter als Olefine mit einer innenständigen Doppelbindung, z.B. But-2-en. Ferner lassen sich Diolefine und Cycloolefine nach dem erfindungsgemäßen Verfahren umsetzen. Allgemein gilt, daß sich das Verfahren auf sämtliche bekannten Fälle anwenden läßt und hierbei die erfindungsgemäße Verbesserung bedingt, so daß sich nähere Angaben hierüber erübrigen.

Dies trifft auch auf die Art der Carbonsäuren zu, wobei Fettsäuren mit 2-6 C-Atomen von besonderem Interesse sind. Sofern diese Säuren wieder abgespalten werden sollen, werden Propionsäure und vor allem Essigsäure aus wirtschaftlichen und verfahrenstechnischen Gründen bevorzugt.

Man kann die Umsetzung in einem Lösungsmittel vornehmen, bevorzugt jedoch in aller Regel die Carbonsäuren selbst und die hieraus gebildeten Ester als flüssiges Reaktionsmedium. Zweckmäßigerweise richtet man es so ein, daß während der Reaktion 0,5 bis 20 mol der Carbonsäure pro Mol des Olefins zur Verfügung stehen und daß die Olefinkonzentration im Reaktionsgemisch zwischen etwa 2 und 25 Gew.-% gehalten wird. Zur Erhöhung der Selektivität bezüglich der Glykolester empfiehlt es sich im allgemeinen, den Olefinumsatz zu begrenzen. Die wirtschaftlich optimalen Umsätze liegen je nach den Reaktionsbedingungen zwischen 5 und 30 %, wobei das erfindungsgemäße Verfahren eine Anhebung um etwa 1-5 Prozentpunkte gestattet. verglichen mit den herkömmlichen Arbeitsweisen.

Die Konzentration des Tellurs im Reaktionsgemisch beträgt

0010282

Vorzugsweise 0,001-10 Gew.-%, besonders 0,1 bis 0,9 Gew.-%.
Das Tellur kann in Form seiner Salze oder, da sich unter
den Reaktionsbedingungen die carbonsauren Salze bilden,
als Metall oder Telluroxid eingesetzt werden.

Die Konzentration des Jods im Reaktionsgemisch kann zwischen
0,1 und 10 Gew.-% liegen, beträgt vorzugsweise jedoch 1 bis
10 Gew.-%. Anstelle von elementarem Jod kann man auch Jodide einsetzen, da aus diesen das Jod unter den Reaktionsbedingungen in Freiheit gesetzt wird.

Vorzugsweise nimmt man die Umsetzung zwischen 120 und 220°C,
insbesondere zwischen 150 und 190 °C unter einem Druck von
1-300, vor allem 10-100 bar vor. Der Sauerstoffpartialdruck
beträgt hierbei vorzugsweise 0,1 bis 20 %, besonders 1 bis
10 % des Gesamtdruckes.

Da die Wasserkonzentration erfindungsgemäß möglichst gering,
mindestens aber unter 1, vorzugsweise 0,8 Gew.-% des Reaktionsgemischs gehalten werden soll, empfiehlt es sich, wasserfreie Ausgangsmaterialien einzusetzen. Im Falle der Essigsäure, die in technischen Qualitäten meistens noch 0,01 bis
0,2 Gew.-% Wasser enthält, kann der Wassergehalt durch Mitverwendung entsprechender Mengen Essigsäureanhydrid erniedrigt werden. Das bei der Reaktion entstehende Wasser
- die Umsetzung von Monoester zum Diester verläuft unter
Wasserabspaltung -kann entweder von wasserentziehenden
Mitteln wie Essigsäureanhydrid abgefangen oder mit den abströmenden Gasen abgeführt werden.

Sieht man von der erfindungsgemäßen Maßnahme ab, kann die
Umsetzung und auch die Aufarbeitung der Reaktionsgemische
auf die Verfahrensprodukte nach den hierfür bekannten Techniken vorgenommen werden.

Durch die erfindungsgemäße Maßnahme wird nicht nur die Selektivität der Reaktion, d.h. die auf den Olefinumsatz bezogenen Ausbeuten an den gewünschten Estern, erheblich verbessert, sondern entgegen jeglicher Erwartung auch die Reaktionsgeschwindigkeit, wie durch die verbesserten Werte für den Olefinumsatz und damit für die Raum-Zeit-Ausbeute zum Ausdruck kommt.

## Beispiel

In einer Versuchsapparatur wurden pro Minute 0,4 Nl Sauerstoff und 4,0 Nl Propylen bei 180 °C unter intensivem Rühren in eine Lösung aus 500 g Essigsäure, 25,25 g Jod und 3,35 g Tellurdioxid eingeleitet. Der mit der Essigsäure vorgelegte Wassergehalt von 6 Gew.-% des Reaktionsgemisches wurde während der Versuchsdauer von 2 Stunden durch Entfernung des zusätzlich gebildeten Reaktionswassers mit dem Gasstrom konstant gehalten, wobei der Wassergehalt durch laufende Probenahme gaschromatographisch ermittelt wurde.

Die ebenfalls gaschromatographisch ermittelten Versuchsergebnisse sind der folgenden Tabelle zu entnehmen.

| Vers.Nr. | Wassergehalte Gew.-% | Propylenumsatz % | Selektivität % |
|----------|---------------------|------------------|----------------|
| zum Vergleich | | | |
| 1 | 20 | 21,8 | 56,3 |
| 2 | 10 | 20,6 | 65,1 |
| 3 | 7,5 | 16,2 | 68,9 |
| 4 | 5 | 15,2 | 76,1 |
| 5 | 2,5 | 14,2 | 81,1 |
| 6 | 2,0 | 13,6 | 82,1 |
| 7 | 1,5 | 13,0 | 83,4 |
| 8 | 1,0 | 12,2 | 83,7 |
| erfindungsgemäß | | | |
| 9 | 0,5 | 13,4 | 85,3 |
| 10 | 0,1 | 14,0 | 90,6 |
| 11 [++] | 0 [++] | 17,0 | 91,6 |

[+] Ausbeuten an Ester, bezogen auf umgesetztes Propylen

[++] anstelle von Essigsäure wurde hier ein Gemisch aus 80 Gew.-% Essigsäure und 20 Gew.-% Essigsäureanhydrid verwendet; im Reaktionsgemisch war kein Wasser nachweisbar.

Wie die Tabelle zeigt, erhöht sich nicht nur die Selektivität mit abnehmender Wasserkonzentration sondern auch der Umsatz.

0010282

## Patentansprüche

1. Verfahren zur Herstellung von Carbonsäureestern vicinaler Glykole durch Oxidation von Olefinen mit Sauerstoff und in Gegenwart von Carbonsäuren sowie von Halogen und von Tellursalzen, dadurch gekennzeichnet, daß man
   - als Halogen Jod verwendet und
   - die Konzentration des Wassers im Reaktionsgemisch unter 1 Gew.-% hält.

2. Verfahren nach Anspruch 1, dadurch gekennzeichnet, daß man es auf die Herstellung von Mono- und Diacetaten des Äthylen- bzw. Propylenglykols durch Umsetzung von Äthylen bzw. Propylen in Gegenwart von Essigsäure anwendet.

3. Verfahren nach Anspruch 2, dadurch gekennzeichnet, daß man den Wassergehalt dadurch auf ein Minimum herabsetzt, indem man Essigsäureanhydrid als wasserentziehendes Mittel mitverwendet.

BAD ORIGINAL

| Kategorie | EINSCHLÄGIGE DOKUMENTE | | KLASSIFIKATION DER ANMELDUNG (Int.Cl.) 3 |
|---|---|---|---|
| | Kennzeichnung des Dokuments mit Angabe, soweit erforderlich, der maßgeblichen Teile | betrifft Anspruch | |
| | DE - A1 - 2 632 158 (CHEM SYSTEMS)<br>+ Gesamt + | 1,2 | C 07 C 67/05<br>C 07 C 69/16 |
| | -- | | |
| | DE - A1 - 2 633 785 (MITSUBISHI CHEMICAL)<br>+ Ansprüche 1 bis 7; Beispiel 21 + | 1,2 | |
| | -- | | |
| | DE - A1 - 2 356 389 (RÖHM GMBH)<br>+ Beispiele + | 1-3 | RECHERCHIERTE SACHGEBIETE (Int. Cl.) 3 |
| | ---- | | C 07 C 67/00<br>C 07 C 69/00 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patent-familie, übereinstimmendes Dokument

| X | Der vorliegende Recherchenbericht wurde für alle Patentansprüche erstellt. |
|---|---|

| Recherchenort | Abschlußdatum der Recherche | Prüfer |
|---|---|---|
| WIEN | 21-12-1979 | KÖRBER |

EPA form 1503.1   06.78